# EUROPEAN PATENT SPECIFICATION

(11) **EP 1 663 121 B1**
(45) Date of publication and mention of the grant of the patent: **13.08.2008**
(21) Application number: 04764580.9
(22) Date of filing: 27.08.2004
(51) Int. Cl.: A61K 8/365, A61K 8/67, A61Q 5/06

(54) **METHOD OF STRAIGHTENING HAIR WITH COMPOSITIONS COMPRISING AN ALPHA-HYDROXY ACID AND A REDUCING AGENT**
VERFAHREN ZUR GLÄTTUNG VON HAAREN MIT ZUSAMMENSETZUNGEN MIT EINER ALPHA-HYDROXY-SÄURE UND EINEM REDUKTIONSMITTEL
COMPOSITION COSMETIQUE POUR DEFRISER LES CHEVEUX COMPRENANT UN ALPHA-HYDROXY ACIDE ET UN REDUCTEUR

(30) Priority: 15.09.2003 EP 03255745
(43) Date of publication of application: 07.06.2006
(73) Proprietor: Unilever PLC, London Greater London EC4P 4BQ (GB); Unilever N.V., 3013 AL Rotterdam (NL)
(72) Inventor: TIWARI, Laxmikant, Highfield, Southampton, SO17 1UE (GB)
(74) Representative: Tansley, Sally Elizabeth
(86) International application number: PCT/EP2004/009606
(87) International publication number: WO 2005/025524

(56) References cited:
- US-A- 5 196 029
- US-B1- 6 482 808
- US-B1- 6 488 920

## Description

### FIELD OF THE INVENTION

The invention relates to hair treatment methods. The methods are particularly suitable to style hair and in particular to lengthen hair.

### BACKGROUND AND PRIOR ART

For centuries long hair has been a desirable attribute. To achieve this straightening devices are sold that mechanically lengthen and straighten the hair, a selection of such devices are disclosed in EP 0 511 892 and WO12/32381 An alternative to the above mechanical approach to lengthen the hair is to stimulate hair growth using chemical hair growth stimulants, such products are disclosed in EP 0 897 712 and WO92/07877.

Skin care compositions comprising ascorbic acid and V-hydroxy acids are described in WO 02/055054, WO 01/066105 and US 5 629 004.

The use of ascorbic acid as a neutralising formulation for hair has been disclosed in US 5 051 252.

US 6 482 808 discloses compositions for restructuring keratin comprising ascorbic acid/salts, ∀-hydroxy acids are used to produce the free acids. The present application discloses processes for lengthening hair. The invention has the further advantages that it prevents the hair frizzing and increasing in volume.

A further advantage of the present invention is that the composition used in the claimed process is stable on storage.

### DESCRIPTION OF THE INVENTION

The present invention provides a method of lengthening hair comprising the step of applying to the hair a non-oxidative hair treatment composition comprising:
i) an α -hydroxy acid, its salt or mixtures thereof and;
ii) a non-thio based reducing agent,
wherein the method does not irreversibly alter the structure of the hair.

### Detailed Description of the Invention

The present invention is based on the finding that reducing agent and alpha-hydroxy acid combinations are particularly useful in styling hair, lengthening hair and increasing high humidity style retention.

In the context of the present invention the term non-oxidative means that the composition does not include ingredients that oxidise the hair, in particular the composition does not contain agents that perm the hair (irreversibly alter the structure) or irreversibly colour the hair.

### Reducing agents

Compositions of the invention comprise a reducing agent, preferably the reducing agent is capable of reducing a disulphide bond.

Preferred reducing agents are antioxidants, in particular antioxidants having the following structure: 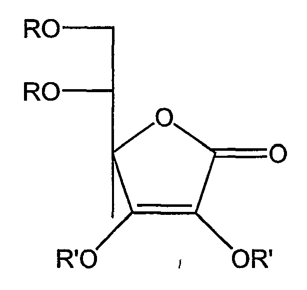 where R is H, -COR" or R" , such that R" is a C1-C18 saturated or unsaturated alkyl chain optionally substituted with COOH groups, and R' is H, a metal ion, phosphate or cation. Preferably R is H.

Particularly preferred cations are metal ions, in particular Ca²⁺, Mg²⁺, and Na⁺, K⁺, and NN₄⁺.

Preferred antioxidants are calcium ascorbate, magnesium ascorbate, ammonium ascorbate, ascorbyl 2-phosphate, niacinamide ascorbate, 6-O-stearoyl-L-ascorbic acid, 6-O-palmitoyl-L-ascorbic acid or mixtures thereof. Ascorbic acid, isoascorbic acid, sodium ascorbate or mixtures thereof are particularly preferred.

The Reducing agents may be in the form of a salt. The cosmetically acceptable salts of the reducing agents of formula (I) are salts, which are non-toxic to humans in the context of the uses according to the invention. The nature of the salt will depend on the acidic or basic groups present in the reducing agents which will depend, in turn, on their precise structural formula. Suitable acid addition salts include, for example, hydrochlorides, phosphate, carboxylates (including acetates, citrates, tartrates, malates, malonates, maleates, lactates, succinates, and fumarates). Suitable base salts include, for example, ammonium salts and alkali metal salts (such as sodium and potassium salts). Suitable salts can be obtained by methods well known to those skilled in the art.

Reducing agents may be used in the present invention in substantially pure form, in the form of unpurified natural extracts, or as a mixture of substantially pure form and natural extract.

Reducing agents may be used in the present invention singly or together with one or more other different reducing agents.

Thio-based reducing agents are absent from the formulation.

If the reducing agent is optically active it is preferred if it is in the L-form.

The hair treatment composition of the invention preferably comprises from 0.1 to 20 wt%, more preferably from 0.2 to 8 wt%, most preferably from 0.5 to 5 wt% of reducing agent in the total formulation.

### α-Hydroxy acid

The formulations of the invention comprise an α -hydroxy acid. The hydroxy acid and/or its salt is preferably a bis (α -hydroxy acid) and/or its salt.

The α -hydroxy acid can comprise one or more carboxylic acid groups, at least one of these carboxylic acid groups should have an α -hydroxy group.

It is particularly preferred an α -hydroxy acid and/or its salt if optically active is in the L-form such as those derived from natural sources, particularly preferred α-hydroxy acid are citric acid and tartaric acid and/or their salts.

The amount of α -hydroxy acid is preferably from 0.1 to 20 wt%, more preferably 0.2 to 8 wt%, most preferably 0.5 to 5 wt% in the total formulation.

The total amount of the reducing agent and α -hydroxy acid in hair treatment compositions of the invention is generally from 0.2 to 40 wt%, preferably from 1 to 10 wt%, more preferably from 2 to 5 wt%.

The weight ratio of reducing agent to α -hydroxy is preferably from 1:0.001 to 0.001:1, preferably from 1:0.01 to 0.01:1, most preferably from 3:1 to 1:3.

### Surfactant

The composition of the invention can optionally include surfactants, which can be included in an amount ranging from 0.1 to 8, preferably from 1 to 4 wt%. of the total composition.

The surfactant may be cationic, anionic, amphoteric or nonionic surfactants.

Cationic surfactants useful in compositions of the invention contain amino or quaternary ammonium hydrophilic moieties which are positively charged when dissolved in the aqueous composition of the present invention. These cationic compounds are commonly included as conditioning compounds.

Examples of suitable cationic surfactants are those corresponding to the general formula:

[N (R₁) (R₂) (R₃) (R₄)]⁺ (X)⁻

in which R₁, R₂, R₃, and R₄ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

Examples of suitable cationic surfactants include: quaternary ammonium chlorides, e.g. alkyltrimethylammonium chlorides wherein the alkyl group has from about 8 to 22 carbon atoms, for example octyltrimethylammonium chloride, dodecyltrimethylammonium chloride, stearyl trimethyl ammonium chloride, behenyl trimethyl ammonium chloride, hexadecyltrimethyl-ammonium chloride, cetyltrimethylammonium chloride, octyldimethylbenzylammonium chloride, decyldimethylbenzyl-ammonium chloride, stearyldimethylbenzylammonium chloride, didodecyldimethylammonium chloride, dioctadecyldimethylammonium chloride, tallow trimethylammonium chloride, cocotrimethylammonium chloride, and the corresponding salts thereof, e.g., bromides, hydroxides. Cetylpyridinium chloride or salts thereof, e.g., chlorideQuaternium -5, Quaternium -31, Quaternium -18 and mixtures thereof.

Preferred are stearyl trimethyl ammonium chloride, hardened tallow trimethyl ammonium chloride, particularly preferred cationic surfactants are cetrimonium chloride, behenyl trimethyl ammonium chloride, di hardened-tallow dimethyl ammonium chloride, distearyl dimethyl ammonium chloride, N,N-bis(2-hydroxyethyl)methyl octadecenyl ammonium chloride or mixtures thereof.

In the compositions of the invention, the level of cationic surfactant is preferably from 0.05 to 12, more preferably 0.1 to 8, most preferably 0.2 to 5 wt% of the total composition.

Examples of amphoteric and zwitterionic surfactants include betaines, glycinates, hydroxysultaines, taurates and glutamates. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred surfactant is a nonionic surfactant, which can be included in an amount ranging from 0 to 8, preferably from 2 to 5 wt%.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Further nonionic surfactants which can be included in shampoo compositions of the invention are the alkyl polyglycosides (APGs) .

### Fatty Materials

Compositions of the invention preferably additionally comprise fatty materials. The combined use of fatty materials and cationic surfactants in conditioning compositions is believed to be especially advantageous, because this leads to the formation of a structured phase, in which the cationic surfactant is dispersed.

By "fatty material" is meant a fatty alcohol, an alkoxylated fatty alcohol, a fatty acid or a mixture thereof.

Preferably, the alkyl chain of the fatty material is fully saturated.

Representative fatty materials comprise from 8 to 22 carbon atoms, more preferably 16 to 22. Examples of suitable fatty alcohols include cetyl alcohol, stearyl alcohol and mixtures thereof. The use of these materials is also advantageous in that they contribute to the overall conditioning properties of compositions of the invention.

Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty alcohol material in conditioners of the invention is suitably from 0.01 to 15, preferably from 0.1 to 10, and more preferably from 0.1 to 5 wt%. The weight ratio of cationic surfactant to fatty alcohol is suitably from 10:1 to 1:10, preferably from 4:1 to 1:8, optimally from 1:1 to 1:7, for example 1:3.

### Suspending Agents

In a preferred embodiment, the hair treatment composition, especially if it is a shampoo composition, further comprises from 0.1 to 5 wt% of a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 941 and Carbopol 980.
An example of a suitable copolymer of a carboxylic acid containing a monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

### Silicone Conditioning Agents

The compositions of the invention can contain, emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the shampoo compositions of the invention will typically have an average silicone droplet size in the composition of less than 30, preferably less than 20, more preferably less than 10 µm, ideally from 0.01 to 1 µm. Silicone emulsions having an average silicone droplet size of ≤ 0.15 µm are generally termed microemulsions.

Examples of suitable pre-formed emulsions include emulsions DC2-1766, DC2-1784, DC-1785 DC-1786 and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones are amino functional silicones.

Also suitable are quaternary silicone polymers are described in EP-A-0 530 974. A preferred quaternary silicone polymer is K3474, ex Goldschmidt.

Also suitable are emulsions of amino functional silicone oils with non-ionic and/or cationic surfactant. Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions DC2-7224, DC2-8467, DC2-8177 and DC2-8154 (all ex Dow Corning).

The total amount of silicone is preferably from 0.01 to 10 %wt of the total composition more preferably from 0.3 to 5, most preferably 0.5 to 3 wt% is a suitable level.

### (ii) Non-silicone Oily Conditioning Components

Compositions according to the present invention may also comprise a dispersed, non-volatile, water-insoluble oily conditioning agent.

By "insoluble" is meant that the material is not soluble in water (distilled or equivalent) at a concentration of 0.1% (w/w), at 25°C.

Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C₂-C₆ alkenyl monomers.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and triesters of glycerol and long chain carboxylic acids such as C₁-C₂₂ carboxylic acids. Preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3 wt%.

In hair treatment compositions containing a conditioning agent, it is preferred that a cationic polymer also be present.

### Styling compound

In some aspects of this invention it is desirable if the composition comprises an additional styling aid.

Particularly useful as styling aids with this invention are hair styling polymers. Hair styling polymers are well known articles of commerce and many such polymers are available commercially which contain moieties, which render the polymers cationic, anionic, amphoteric or nonionic in nature. The polymers may be synthetic or naturally derived.

The amount of the hair styling polymer may range from 0.1 to 10%, preferably 0.5 to 8 %, more preferably 0.75 to 6% by weight based on total weight of the composition.

### Further components

An aqueous phase thickener is preferably present and can be based on cellulose derivative, in particular hydroxyethyl cellulose or cetyl hydroxyethyl cellulose. An alternative aqueous phase thickener is carbomer. Such aqueous phase thickeners are typically present in an amount from 0.01% to 10% by weight.

Hair care compositions of the present invention can comprise a carrier, or a mixture of such carriers, which are suitable for application to the hair. The carriers are present at from about 0.5% to about 99.5%, preferably from about 5.0% to about 99.5%, more preferably from about 10.0% to about 98.0%, of the composition. As used herein, the phrase "suitable for application to hair" means that the carrier does not damage or negatively affect the aesthetics of hair or cause irritation to the underlying skin.

Compositions according to the invention comprise a buffer or pH adjuster. Preferred buffers or pH adjusters include weak acids and bases such as glycine/sodium hydroxide, citric acid, triethanolamine, lactic acid, succinic acid, acetic salt and salts thereof. Frequently a mixture of buffering system is used such as sodium citrate and citric acid.

Carriers suitable for use with hair care compositions of the present invention include, for example, those commonly used in creams. The carriers used herein can include a wide range of components conventionally used in hair care compositions. The carriers can contain a solvent to dissolve or disperse the styling compound being used, with water, the C₁-C₆ alcohols, lower alkyl acetate and mixtures thereof being preferred. The carriers can also contain a wide variety of additional materials such as acetone, hydrocarbons (such as isobutane, hexane, decene), water, ethanol, volatile silicone derivatives, and mixtures thereof. The solvents used in such mixtures may be miscible or immiscible with each other.

Further general ingredients suitable for all product forms include, sun-screening agents, preservatives, anti-oxidants, anti-dandruff actives, and emulsifiers for emulsifying the various carrier components of the compositions of the invention.

The compositions of the present invention may also contain adjuncts suitable for hair care. Generally such ingredients are included individually at a level of up to 2, preferably up to 1 wt% of the total composition. Suitable hair care adjuncts, include amino acids, sugars and ceramides.

Compositions of the present invention are formulated into hair care compositions, especially products with hair styling claims. The compositions are for use in styling human hair and, more preferably, they are packaged and labeled as such. The final product form of hair treatment compositions according to the invention may suitably be, for example, shampoos, conditioners, sprays, mousses, gels, waxes or lotions. Particularly preferred product forms are leave in products particularly creams and gels.

It is preferred if the products are left on hair after application and not immediately washed off.

The composition preferably has a pH from 2.5 to 8, more preferably 3 to less than 7, most preferably from 3.2 to 6. If the composition is a solid, the above pH ranges apply to a 10% aqueous solution of the composition at 25°C.

The following non-limiting Examples further illustrate the preferred embodiments of the invention. All percentages referred to in the examples and throughout this specification are by weight based on total weight unless otherwise indicated.

Examples of the invention are illustrated by a number, comparative examples are illustrated by a letter.

### Examples

| Chemical Name | %.w/w | Example | | | | | |
|---|---|---|---|---|---|---|---|
| | **A** | **1** | **2** | **3** | **4** | **5** | **B** |
| Cetrimonium Chloride - CTAC | 2.10 | 2.10 | 2.10 | 2.10 | 2.10 | 2.10 | 2.10 |
| Cetyl Alcohol -CSA | 4.00 | 4.00 | 4.00 | 4.00 | 4.00 | 4.00 | 4.00 |
| Glyceryl Stearate | 0.48 | 0.48 | 0.48 | 0.48 | 0.48 | 0.48 | 0.48 |
| Methyl Paraben | 0.16 | 0.16 | 0.16 | 0.16 | 0.16 | 0.16 | 0.16 |
| Mineral oil | 0.25 | 0.25 | 0.25 | 0.25 | 0.25 | 0.25 | 0.25 |
| Petroleum Jelly | 0.16 | 0.16 | 0.16 | 0.16 | 0.16 | 0.16 | 0.16 |
| Glycerine | 0.80 | 0.80 | 0.80 | 0.80 | 0.80 | 0.80 | 0.80 |
| Hydroxyethyl Cellulose | 0.12 | 0.12 | 0.12 | 0.12 | 0.12 | 0.12 | 0.12 |
| Dimethicone Emulsion (50%) | 2.10 | 2.10 | 2.10 | 2.10 | 2.10 | 2.10 | 2.10 |
| DMDM Hydantoin | 0.24 | 0.24 | 0.24 | 0.24 | 0.24 | 0.24 | 0.24 |
| Tartaric Acid | | 2.00 | 1.00 | 0.20 | | | |
| Citric acid | | | | | 1.00 | 0.02 | |
| Na Ascorbate | | 2.00 | 1.00 | 2.00 | 1.00 | 2.00 | 2.00 |
| NaOH (2.5M) | tc pH 3.8 80 | | | | | | |
| Water, perfume and minors | to 100% % | | | | | | |

The examples were repeated at pH 7.0

### Experimental Methodology

Set of swatches of hair were made from naturally curly hair. The initial length of all the swatches were measured before the treatment by taking a photograph and using a ruler (L₀).

The swatches were cleaned using a base shampoo. The treatment protocol was as follows. All swatches were treated twice 10%w/w of a shampoo by massaging the shampoo into the hair for 30 seconds and then rinsing for 30 seconds. Thereafter, 20%w/w of a conditioner was applied by massaging it to the hair for 60 seconds, left for 90 seconds and then rinsing it for 60 seconds. Thereafter, the switches were treated with Examples 1 and A by massaging with 10%w/w for 3 minutes and then combed and left to dry at 20°C, 50RH for 3hours. At this stage the length of the swatches were measured (L₁).

The switches were then exposed to 30°C, 80RH for 3hours.
A final measurement of the length of the swatches was made.

| **Formulation** | **% Lengthening after drying @20°C, 50RH** | **% Lengthening after exposure @30°C, 80RH** |
|---|---|---|
| Example A | 0 | 0 |
| Example 1 | 5.54 | 1.0 |

The Examples were placed in sealed test tubes and the stored at room temperature for 17 days. On storage the Examples made at pH 7.0 had a very pungent odour, stronger than those at pH 3.8.

## Claims

1. A method of lengthening hair comprising the step of applying to the hair a non-oxidative hair treatment composition comprising:
i)an α -hydroxy acid, its salt or mixtures thereof and;
ii)a non-thio based reducing agent, wherein the method does not irreversibly alter the structure of the hair.

2. A method according to claim 1 in which the non-thio based reducing agent of the hair treatment composition is an antioxidant.

3. A method according to claim 1 or claim 2 in which the antioxidant of the hair treatment composition has the following structures: 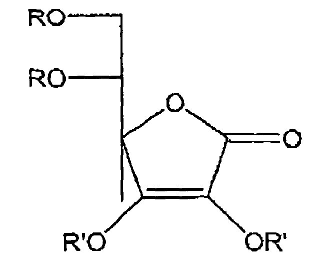 where R is H, -COR" or R" , such that R" is a C1-C18 saturated or unsaturated alkyl chain optionally substituted with COOH groups and R' is H, a metal ion, phosphate or cation.

4. A method according to any preceding claim in which the reducing agent of the hair treatment composition is selected from ascorbic acid, iso-ascorbic acid, sodium ascorbate or mixtures thereof.

5. A method according to any preceding claim in which the α -hydroxy acid and/or its salt i) is a bis (α -hydroxy acid) and/or its salt.

6. A method according to any preceding claim in which the an α -hydroxy acid i) is tartaric acid or its salt.

7. A method according to any preceding claim in which the an α -hydroxy acid and/or its salt if optically active is in the L-form.

8. A method according to any preceding claim in which the composition has a pH from 3 to below 7.

9. A method according to any preceding claim in which the total amount of α -hydroxy acid acid/salt i) is from 0.1 to 20 wt% of the total composition.

10. A method according to any preceding claim in which the total amount reducing agent ii) is from 0.1 to 20 wt% of the total composition.

11. A method according to any preceding claim in which the hair treatment composition further comprises a cationic or silicone based conditioning agent.

12. A method according to any preceding claim in which the hair treatment composition further comprises a styling agent.

13. A method according to any preceding claim in which the hair treatment composition comprises an aqueous base.

## Patentansprüche

1. Verfahren zur Verlängerung von Haar, umfassend den Schritt eines Auftragens einer nicht-oxidativen Haarbehandlungszusammensetzung, umfassend:
i) eine α-Hydroxysäure, ihr Salz oder Gemische davon und
ii) ein nicht-Thio-basiertes Reduktionsmittel,
auf das Haar, wobei das Verfahren die Struktur des Haars nicht irreversibel verändert.

2. Verfahren nach Anspruch 1, wobei das nicht-Thio-basierte Reduktionsmittel der Haarbehandlungszusammensetzung ein Antioxidans ist.

3. Verfahren nach Anspruch 1 oder Anspruch 2, wobei das Antioxidans der Haarbehandlungszusammensetzung die folgenden Strukturen hat: 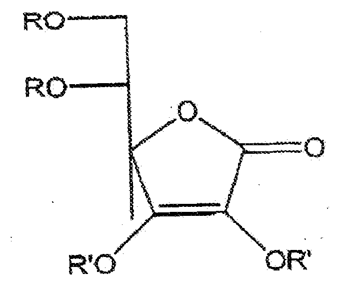 worin R H, -COR" oder R" ist, so dass R" eine gesättigte oder ungesättigte C₁-C₁₈-AlkylKette, die gegebenenfalls mit COOH-Gruppen substituiert ist, und R' H, ein Metallion, Phosphat oder ein Kation ist.

4. Verfahren nach einem vorangehenden Anspruch, wobei das Reduktionsmittel der Haarbehandlungszusammensetzung aus Ascorbinsäure, Isoascorbinsäure, Natriumascorbat und Gemischen davon ausgewählt wird.

5. Verfahren nach einem vorangehenden Anspruch, wobei die α-Hydroxysäure und/oder ihr Salz i) eine Bis(α-hydroxysäure) und/oder ihr Salz ist.

6. Verfahren nach einem vorangehenden Anspruch, wobei die α-Hydroxysäure i) Weinsäure oder ihr Salz ist.

7. Verfahren nach einem vorangehenden Anspruch, wobei die α-Hydroxysäure und/oder ihr Salz, wenn sie/es optisch aktiv ist, in der L-Form ist.

8. Verfahren nach einem vorangehenden Anspruch, wobei die Zusammensetzung einen pH von 3 bis unter 7 hat.

9. Verfahren nach einem vorangehenden Anspruch, wobei die Gesamtmenge an α-Hydroxysäure/-salz i) 0,1 bis 20 Gew.-% der gesamten Zusammensetzung ist.

10. Verfahren nach einem vorangehenden Anspruch, wobei die Gesamtmenge an Reduktionsmittel ii) 0,1 bis 20 Gew.-% der gesamten Zusammensetzung ist.

11. Verfahren nach einem vorangehenden Anspruch, wobei die Haarbehandlungszusammensetzung außerdem ein kationisches oder Silicon-basiertes Konditionierungsmittel umfasst.

12. Verfahren nach einem vorangehenden Anspruch, wobei die Haarbehandlungszusammensetzung außerdem ein Styling-Mittel umfasst.

13. Verfahren nach einem vorangehenden Anspruch, wobei die Haarbehandlungszusammensetzung eine wässrige Base umfasst.

## Revendications

1. Procédé d'élongation des cheveux comprenant l'étape d'application sur les cheveux d'une composition de traitement capillaire non oxydante comprenant :
i) un α-hydroxy acide, son sel ou des mélanges de ceux-ci ; et
ii) un agent de réduction sans groupe thio, dans lequel le procédé n'altère pas de manière irréversible la structure des cheveux.

2. Procédé selon la revendication 1, dans lequel l'agent de réduction sans groupe thio de la composition de traitement capillaire est un antioxydant.

3. Procédé selon la revendication 1 ou 2, dans lequel l'antioxydant de la composition de traitement capillaire a la structure suivante : 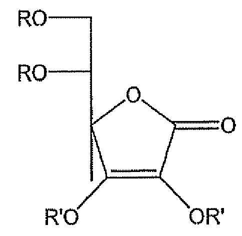 où R est H, -COR" ou R", de sorte que R" soit une chaîne alkyle saturée ou insaturée en C₁-C₁₈ éventuellement substituée par des groupes COOH, et R' est H, un ion métallique, un phosphate ou un cation.

4. Procédé selon l'une quelconque des revendications précédentes, dans lequel l'agent de réduction de la composition de traitement capillaire est choisi parmi l'acide ascorbique, l'acide iso-ascorbique, l'ascorbate de sodium ou des mélanges de ceux-ci.

5. Procédé selon l'une quelconque des revendications précédentes, dans lequel l'α-hydroxy acide et/ou son sel i) est un bis(α-hydroxy acide) et/ou son sel.

6. Procédé selon l'une quelconque des revendications précédentes, dans lequel l'α-hydroxy acide
i) est l'acide tartrique ou son sel.

7. Procédé selon l'une quelconque des revendications précédentes, dans lequel l'α-hydroxy acide et/ou son sel, s'il est optiquement actif, est sous la forme L.

8. Procédé selon l'une quelconque des revendications précédentes, dans lequel la composition a un pH de 3 à moins de 7.

9. Procédé selon l'une quelconque des revendications précédentes, dans lequel la quantité totale d'α-hydroxy acide/sel i) est de 0,1 à 20 % en poids de la composition totale.

10. Procédé selon l'une quelconque des revendications précédentes, dans lequel la quantité totale de l'agent de réduction ii) est de 0,1 à 20 % en poids de la composition totale.

11. Procédé selon l'une quelconque des revendications précédentes, dans lequel la composition de traitement capillaire comprend en outre un agent conditionneur cationique ou à base de silicone.

12. Procédé selon l'une quelconque des revendications précédentes, dans lequel la composition de traitement capillaire comprend en outre un agent coiffant.

13. Procédé selon l'une quelconque des revendications précédentes, dans lequel la composition de traitement capillaire comprend une base aqueuse.
